# FASCICULE DE BREVET EUROPEEN

(11) **EP 2 737 052 B1**
(45) Date de publication et mention de la délivrance du brevet: **27.06.2018**
(21) Numéro de dépôt: 12744100.4
(22) Date de dépôt: 24.07.2012
(51) Int. Cl.: C12N 5/00, G01N 33/50, C12M 3/00, A61F 2/02

(54) **SUPPORT D'ADHESION MICROSTRUCTURÉ DE CELLULES ANIMALES OU HUMAINES**
MIKROSTRUKTURIERTES HAFTSUBSTRAT FÜR TIER- ODER MENSCHLICHE ZELLEN
MICROSTRUCTURED ADHESION SUBSTRATE FOR ANIMAL OR HUMAN CELLS

(30) Priorité: 26.07.2011 FR 1156814
(43) Date de publication de la demande: 04.06.2014
(73) Titulaire: Université Joseph Fourier, 38041 Grenoble Cedex 09 (FR); Centre National de la Recherche Scientifique, 75794 Paris Cedex 16 (FR)
(72) Inventeur: BALLAND, Martial, F-38400 Saint Martin D'heres (FR); MANDAL, Kalpana, West Bengal Raiganj 733134 (IN); BUREAU, Lionel, F-38240 Meylan (FR)
(74) Mandataire: Sarlin, Laure V.
(86) Numéro de dépôt international: PCT/FR2012/051755
(87) Numéro de publication internationale: WO 2013/014389

(56) Documents cités:
- EP-A1- 1 600 498
- YAMATO MASAYUKI ET AL: "Nanofabrication for micropatterned cell arrays by combining electron beam-irradiated polymer grafting and localized laser ablation.", JOURNAL OF BIOMEDICAL MATERIALS RESEARCH. PART A 15 DEC 2003 LNKD- PUBMED:14666924, vol. 67, no. 4, 15 décembre 2003 (2003-12-15), pages 1065-1071, XP055022125, ISSN: 1549-3296
- WEDER G ET AL: "The quantification of single cell adhesion on functionalized surfaces for cell sheet engineering", BIOMATERIALS SEPTEMBER 2010 ELSEVIER LTD GBR, vol. 31, no. 25, septembre 2010 (2010-09), pages 6436-6443, XP027102950, DOI: DOI:10.1016/J.BIOMATERIALS.2010.04.068
- MALHAM IBRAHIM B ET AL: "Density effects on collapse, compression, and adhesion of thermoresponsive polymer brushes.", LANGMUIR : THE ACS JOURNAL OF SURFACES AND COLLOIDS 6 APR 2010 LNKD- PUBMED:19961208, vol. 26, no. 7, 6 avril 2010 (2010-04-06), pages 4762-4768, XP055022552, ISSN: 1520-5827 cité dans la demande
- Kalpana Mandal1 ET AL: "Thermoresponsive micropatterned substrates for single cell studies", , 10 novembre 2011 (2011-11-10), pages 1-12, XP055022130, Extrait de l'Internet: URL:http://hal.archives-ouvertes.fr/docs/0 0/64/01/15/PDF/paperPlos1.pdf [extrait le 2012-03-16]

## Description

La présente invention concerne le domaine technique de la biologie cellulaire et des supports adaptés pour mener des analyses de biologie cellulaire. En particulier, la présente invention concerne un support d'adhésion de cellules animales ou humaines, et un procédé de préparation d'un tel support, ainsi qu'un procédé d'analyse mettant en oeuvre un tel support, avec notamment une étape de détachement de la ou des cellules ayant adhérées au support.

A l'heure actuelle, des techniques d'analyse cellulaire automatisées permettant, par exemple, le criblage phénotypique à haut débit de cellules et l'étude de l'activité de molécules sur l'activation ou l'inhibition de fonctions cellulaires, sont très recherchées. Un des objectifs est de pouvoir mener des analyses biologiques précises tout en utilisant des procédés automatisés à haut débit sur un grand nombre de cellules.

Le développement des techniques de micro-structuration de surface a conduit, ces dernières années, à la réalisation de dispositifs permettant de contrôler spatialement l'adhésion entre des cellules et un support solide. La maîtrise de l'espacement et de la géométrie des zones adhésives permet ainsi d'immobiliser sur une surface des cellules individuelles régulièrement réparties et contraintes d'adopter une forme fixée par la géométrie du motif adhésif (EP 1664266 ; Falconnet et al. Biomaterials 27 (2006) pp 3044-3063). Il est ainsi possible d'empêcher les cellules de migrer et d'orienter de la même façon chaque cellule.

Ce type de dispositifs permet d'étudier très précisément les effets de paramètres environnementaux physiques ou biologiques sur la réponse cellulaire, comme le rôle joué par des composés biologiquement actifs dans l'amplification ou l'inactivation de fonctions cellulaires spécifiques. L'intérêt majeur de ces supports adhésifs micro-structurés est qu'ils permettent d'obtenir des informations sur une grande population de cellules placées dans un environnement contrôlé supprimant la variabilité de forme des cellules. On peut ainsi mener des analyses statistiques par le biais de tests automatisés rendus possibles par l'aspect régulier du réseau de motifs adhésifs. Ces dispositifs sont utilisés, par exemple, dans des applications de criblage de médicaments, ou encore dans des études fondamentales visant à déterminer l'impact d'un stimulus environnemental sur l'organisation intracellulaire, l'adhésion, la division ou l'apoptose des cellules.

La fabrication de telles surfaces micro-structurées repose sur l'élaboration, sur un support, d'un revêtement présentant des motifs adhésifs séparés par des régions sur lesquelles les cellules ne peuvent pas adhérer. Plusieurs critères importants doivent être pris en compte quant aux caractéristiques et propriétés de ces supports :
- la résolution spatiale des motifs adhésifs doit être de l'ordre de quelques micromètres, pour des motifs de dimensions adaptés à la taille d'une cellule individuelle.
- il doit exister un fort contraste adhésif entre les motifs et le reste du support, afin de bien contrôler la localisation spatiale des cellules.
- les zones anti-adhésives doivent assurer leur fonction sur des durées compatibles avec les expériences de culture cellulaire : les revêtements doivent donc être stables, lors de leur utilisation, sur des temps allant de quelques heures à plusieurs jours, en présence du milieu liquide nécessaire à la culture des cellules et à une température de 37°C.
- les surfaces doivent être stables et rester fonctionnelles le plus longtemps possible, avant utilisation, dans des conditions de stockage simples, idéalement à l'air et dans des conditions ambiantes de température.

L'élaboration de supports micro-structurés pour la biologie cellulaire repose à l'heure actuelle sur deux techniques principales qui sont l'impression par microcontact (en anglais « microcontact printing ») et la photolithographie, combinée avec diverses méthodes de fonctionnalisation chimique de surface.

Les méthodes utilisant l'impression par microcontact telles que décrites dans le brevet EP 1664266 mettent en jeu la stratégie et les étapes suivantes:
1- fabrication d'une empreinte par moulage d'une résine photosensible dont le relief est le négatif du motif final souhaité.
2- fabrication d'un tampon en élastomère moulé sur l'empreinte précédente.
3- encrage du tampon par mise en contact avec une solution qui contient la première espèce de molécules que l'on souhaite déposer sur la surface du support sous la forme du motif final souhaité.
4- mise en contact directe du tampon avec le support pour le transfert des molécules.
5- adsorption sur le support, dans les zones non couvertes à l'étape 4, d'une deuxième espèce de molécule. Cette étape se fait par exemple par immersion du support tel que modifié à l'étape 4 dans une solution contenant la deuxième espèce moléculaire.

Un exemple de réalisation suivant ce protocole consiste à déposer, à l'étape 4, une protéine de la matrice extracellulaire impliquée dans l'adhésion, comme la fibronectine. La présence de fibronectine selon un motif régulièrement organisé sur le support facilite l'adhésion cellulaire sur ce motif. Pour empêcher l'étalement des cellules en dehors du motif, les zones non couvertes de fibronectine sont couvertes, à l'étape 5, d'un copolymère de type PLL-PEG [poly(L-lysine)-poly(éthylène glycol)], le bloc PEG assurant la fonction anti-adhésive alors que le bloc cationique PLL assure une liaison électrostatique des macromolécules avec le support si celui-ci est chargé négativement.

Des variantes de ce protocole consistent à déposer, à l'étape 4, l'espèce moléculaire réalisant la fonction anti-adhésive, qui peut être par exemple un alkylsilane ou un alcanethiol. Il faut alors utiliser un tampon dont le relief est le négatif du motif adhésif final désiré. L'étape 5 consiste ensuite à adsorber dans les zones restées libres une protéine d'adhésion telle que la fibronectine ou le collagène.

La technique d'impression par microcontact présente un certain nombre d'avantages tels que son caractère versatile, le fait qu'elle permette la création de motifs ayant des dimensions micrométriques, le fait que plusieurs tampons puissent être créés à partir d'un même moule qui peut être utilisé plusieurs fois.

Cependant, la création des moules nécessite l'utilisation d'une salle blanche (pièce ou série de pièces où la concentration particulaire est maîtrisée afin de minimiser l'introduction, la génération, la rétention de particules à l'intérieur). De plus un certain nombre de limitations supplémentaires sont à noter, que sont.
- La déformation du tampon: la mise en contact du tampon avec le support est une étape délicate. En effet, une pression trop forte sur le tampon peut déformer celui-ci et conduire à des différences importantes dans la géométrie et les dimensions des motifs imprimés en comparaison avec les motifs originaux présents sur le moule.
- Contamination du support: durant le processus de cuisson du moule des fragments peuvent ne pas cuire et donc induire des défauts qui vont influencer la qualité du tampon.
- Lors de la cuisson du tampon, le polymère peut se rétracter en taille et donc créer une différence entre la taille des motifs désirés et la taille physique (réelle) de ceux-ci.
- Mobilité de l'encre (molécule servant à créer les motifs): De la diffusion des molécules à l'interface tampon/support peut apparaître pendant l'étape de mise en contact avec le support. Cette mobilité latérale (diffusion) peut induire un étalement des molécules en dehors des régions (motifs) désirés.
- Les deux espèces moléculaires déposées sur le support sont la plupart du temps physisorbées, c'est-à-dire en interaction relativement faible avec le support. Ceci peut poser des problèmes de stabilité du motif adhésif lors de la culture des cellules.

La technique de photolithographie, quant à elle, utilise la lumière, en général dans la gamme de longueur d'onde correspondant aux UV (ultraviolets), pour produire des espèces réactives oxygénées au voisinage de la surface d'un support sur lequel est présent un groupement chimique photosensible, qui va être modifié ou dégradé sous l'effet du rayonnement. Cette modification est spatialement sélective lorsque l'illumination de la surface a lieu au travers d'un photo-masque, utilisé comme "pochoir", présentant des zones transparentes et des régions opaques au rayonnement UV.

Des motifs adhésifs peuvent être obtenus par cette méthode en illuminant, au travers d'un photo-masque portant le motif souhaité, un revêtement uniforme, initialement anti-adhésif, préalablement déposé sur un support. Les modifications chimiques induites vont permettre l'adsorption, sur les zones éclairées, de protéines d'adhésion. La lumière peut être également utilisée pour détacher ou détruire l'espèce moléculaire anti-adhésive, et permettre ainsi le dépôt ultérieur, aux endroits illuminés, de protéines ou de ligands permettant l'adhésion de cellules.

Un exemple de fabrication de supports micro-structurés selon ce procédé repose sur l'adsorption, sur une surface de verre, d'une couche mince de PLL-PEG. Cette couche, une fois débarrassée du solvant utilisé pour le dépôt, est mise en contact (direct ou via un liquide de contact) avec un photo-masque portant le motif souhaité. L'illumination de la surface pendant quelques minutes aux UV profonds (longueur d'onde inférieure à 200 nm) a pour effet de générer des groupements carboxyl sur les macromolécules de PEG. Ces groupements carboxyl permettent alors l'adsorption de fibronectine, qui favorise l'adhésion des cellules aux endroits du revêtement qui ont été insolés (Azioune et al. Lab On a Chip 9 (2009) pp 1640-1642).

La technique de photolithographie est une technique rapide et simple de mise en oeuvre. Elle ne nécessite pas l'utilisation d'une salle blanche. Elle permet de réaliser des motifs adhésifs avec une résolution micrométrique de façon fiable et reproductible. Les photo-masques, qui sont disponibles à façon dans le commerce, sont réutilisables un très grand nombre de fois. Néanmoins, cette technique présente également des problèmes liés à la stabilité des revêtements micro-structurés qui sont le plus souvent formés par simple physisorption d'une espèce moléculaire sur le support.

Par ailleurs, quelle que soit la technique de micro-structuration utilisée, lorsque les analyses réalisées sur les cellules immobilisées sur le support doivent être suivies de tests pratiqués en solution, ce qui est très fréquent, il est indispensable de pouvoir détacher de la surface la population de cellules étudiée, afin de mettre les cellules en suspension. A cet effet il est nécessaire, avec tous les supports micro-structurés actuels, d'avoir recours à des méthodes enzymatiques ou chimiques. On utilise ainsi classiquement la trypsine, enzyme qui dégrade les protéines impliquées dans l'adhésion cellulaire, ou l'acide éthylène diamine tétracétique (EDTA), qui chélate les ions calcium nécessaires au fonctionnement des intégrines, protéines transmembranaires participant à l'adhésion. Ces deux méthodes sont cependant susceptibles d'affecter la viabilité cellulaire, et représentent des méthodes de détachement « agressives» qui peuvent affecter les résultats des tests qui suivent le décollement des cellules.

La réalisation de supports micro-structurés permettant, d'une part, l'étude de cellules adhérées, puis dans un second temps le détachement de celles-ci par une technique moins invasive que l'utilisation de trypsine ou d'EDTA, représenterait une avancée importante.

C'est un des objectifs que se propose d'atteindre la présente invention qui propose un support d'adhésion pour au moins une cellule animale ou humaine définissant une surface sur laquelle est présente au moins un motif d'adhésion pour au moins une cellule animale ou humaine, ledit motif comportant au moins deux zones adhésives à ladite cellule qui sont espacées par une zone non adhésive à ladite cellule sur laquelle un polymère non-adhésif à ladite cellule est immobilisé, caractérisé en ce que le polymère est sélectionné de manière à présenter une conformation qui peut être modifiée par variation d'un paramètre de l'environnement dans lequel se trouve le support. En particulier, le polymère est sélectionné de manière à définir une couche dont l'épaisseur peut être augmentée par rapport à une épaisseur de référence mesurée lorsque le support est placé dans l'eau pure à 37°C et en l'absence de lumière, par modification d'un paramètre de l'environnement dans lequel se trouve le support.

Dans le cadre de l'invention, les zones adhésives d'un motif adhésif sont disposées, de manière telle que la cellule puisse adhérer, dans des conditions d'adhésion, sur les zones adhésives en formant un pont sur le polymère non-adhésif et le polymère non-adhésif se présente sous une conformation dite compactée, dans les conditions d'adhésion de la cellule, pour se déployer dans une conformation dite déployée par modification d'un paramètre de l'environnement dans lequel se trouve le support.

L'invention a également pour objet l'ensemble des supports et procédés tels que définis dans les revendications.

L'invention concerne notamment un procédé de détachement *in vitro* d'au moins une cellule préalablement adhérée sur le ou les motifs adhésifs d'un support selon l'invention dans lequel le détachement de la cellule est réalisé par variation d'un paramètre environnemental dans lequel se trouve le support, entraînant un déploiement du polymère non-adhésif.

La description qui va suivre, en référence aux Figures annexées, permet de décrire l'invention de manière détaillée.

La **Figure 1** présente un schéma de principe permettant d'illustrer comment un dispositif selon l'invention permet de décoller une cellule qui a adhéré sur un motif adhésif, en faisant varier un paramètre environnemental. A gauche, une cellule adhérée sur le support et formant un pont au-dessus d'une zone non adhésive de polymère en conformation dite compactée, également nommée globulaire. A droite, après variation du paramètre environnemental, les macromolécules de polymère adoptent une conformation déployée, également nommée gonflée, ce qui entraine le décollement de la cellule.

La **Figure 2** met en évidence la dépendance de l'épaisseur sèche des brosses de poly(N-isopropylacrylamide) (PNIPAM) obtenues dans les exemples, avec le temps de polymérisation. Concentration initiale en APTES lors de la première étape de greffage : 2.10⁻⁴ M.

La **Figure 3** met en évidence la dépendance de l'épaisseur sèche des brosses de poly(N-isopropylacrylamide) (PNIPAM) obtenues dans les exemples avec le temps d'insolation aux UV profonds.

La **Figure 4** montre (a) des motifs adhésifs en forme de V marqués en fluorescence par adsorption de fibronectine/fibrinogène-alexa fluor observés à 546nm. Taille de l'image : 895x676 µm². (b) des motifs adhésifs en forme de V marqués en fluorescence par adsorption de fibronectine/fibrinogène-alexa fluor observés à 546nm. Insert : profil d'intensité de fluorescence le long de la ligne tracée sur l'image. Taille de l'image : 420x315 µm².

La **Figure 5** montrent des exemples de forme de motifs adhésifs, en forme d'anneau (a) ; de triangle (b) ; de rectangle (c) ; de « pacman » (d) ; marqués en fluorescence par adsorption de fibronectine/fibrinogène-alexa fluor observés à 546nm.

La **Figure 6** est une image en contraste de phase d'un réseau de cellules REF52 adhérées sur des motifs en forme de V. Culture cellulaire effectuée sur un support sur lequel de la fibronectine a été préalablement adsorbée. Taille de l'image 270x270 µm².

La **Figure 7** est une image d'une cellule unique REF52. Le signal de fluorescence, détecté uniformément dans la cellule, correspond au marquage paxilline-YFP.

La **Figure 8** est une image moyenne d'un marquage paxilline-YFP construite à partir de 11 images de cellules REF52 individuelles. L'image montre l'agrégation de la paxilline au niveau des sommets du motif en V sur lequel les cellules ont adhéré. La tache centrale correspond à la position du noyau cellulaire : la forte épaisseur dans cette région entraine une saturation du signal de fluorescence.

La **Figure 9** présente des images en microscopie en champ claire obtenues sur des cultures cellulaires MEF adhérées sur différents motifs et réalisées sans adsorption préalable de ligand. Gauche : triangle enfermant une zone centrale couverte de PNIPAM. Centre : anneau. Droite : rectangle enfermant une zone centrale couverte de PNIPAM.

La **Figure 10** présente des images en champ clair d'une cellule REF52 initialement adhérée sur un motif en V. (a) : immédiatement après une mise à température T=30°C. (b) 36 minutes après l'abaissement de température. (c) 47 minutes après. Taille des images : 85x85 µm².

La **Figure 11** présente des images en champ clair d'une cellule MEF initialement adhérée sur un motif rectangulaire. (a) : immédiatement après une mise à température T=30°C. (b) 10 minutes après l'abaissement de température. (c) 25 minutes après. Taille des images : 55x55 µm².

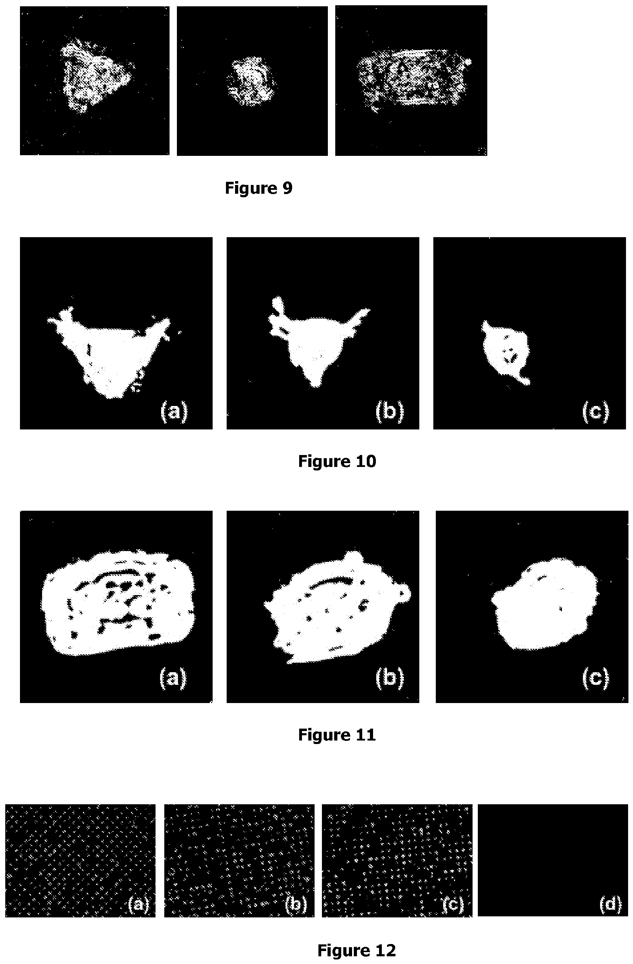

La **Figure 12** présente des images en contraste de phase de réseaux de cellules MEF adhérant sur un support micro-structuré. (a) : première utilisation du support. (b) : deuxième culture de cellules sur le même support, après décollement des cellules de la première culture. (c) : troisième culture de cellules sur le même support, après décollement des cellules de la deuxième culture. Les taches brillantes correspondent aux cellules individuelles adhérées. (d) : état de la surface du support entre deux cultures. Aucune cellule n'est adhérée après mise à température ambiante et rinçage du support. Taille des images : 2200x1664 µm².

Les supports selon l'invention sont conçus pour permettre (i) de faire adhérer une cellule sur un motif adhésif, et (ii) de détacher ensuite cette cellule du support par la variation d'un paramètre environnemental comme la température ou la lumière, sans avoir recours aux méthodes enzymatiques ou chimiques telles que l'emploi de trypsine ou d'EDTA.

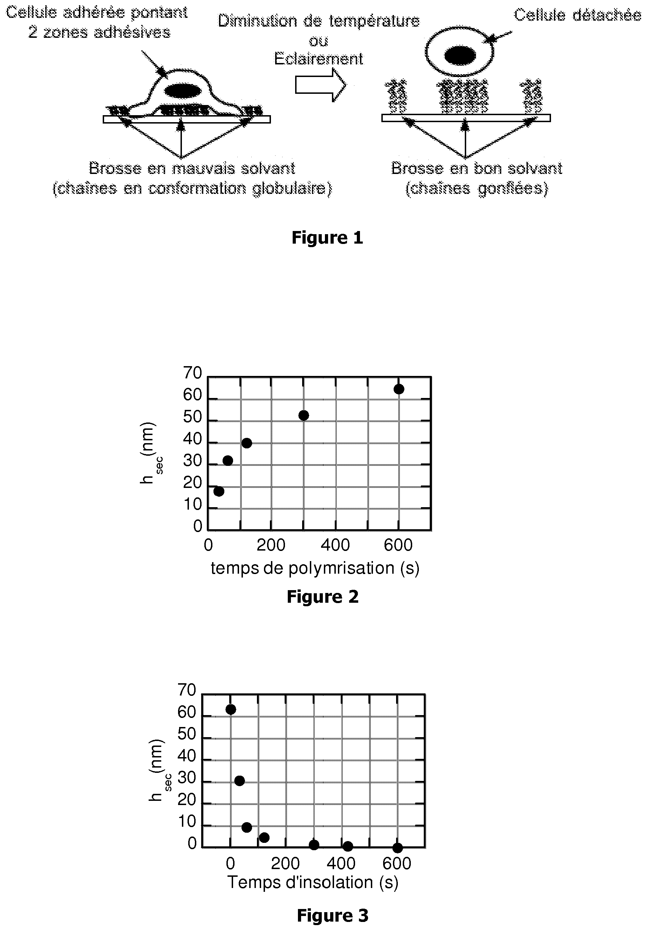

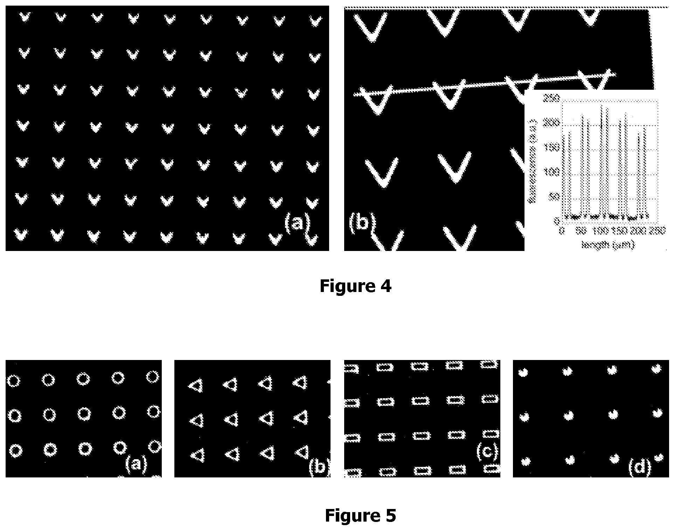

Le motif adhésif présente une forme telle qu'une cellule va pouvoir y adhérer en se liant à des zones dites adhésives, mais en venant se positionner au-dessus d'une zone non adhésive du motif. Le principe est illustré **Figure 1** qui montre une cellule liée aux zones adhésives d'un motif adhésif. Au moins une partie des zones adhésives étant séparées par une zone non adhésive, la cellule une fois adhérée sur les zones adhésives va former un pont sur la zone non adhésive. Les zones adhésives, bien que séparées pour partie au moins, par une zone non adhésive peuvent être connectées entre elles pour former une forme ouverte (cas d'une forme en L, par exemple) ou fermée (cas d'un anneau ou polygone par exemple) ou bien être isolées les unes des autres (cas d'une ligne et un ou deux points, par exemple). Les zones adhésives peuvent être constituées de toute forme bidimensionnelle, et se présenter sous la forme de ligne, bande, point, arc de cercle .... La distance maximale créée par la zone non adhésive séparant deux zones adhésives d'un même motif, est le plus souvent inférieure à 50 µm, de préférence dans la gamme 5 à 20 µm. Les zones adhésives des motifs adhésifs peuvent, notamment, former une ligne brisée ou courbe qui peut être fermée délimitant par exemple un polygone ou un anneau ou qui peut être ouverte sous la forme, par exemple d'un V, U, C ou L ou correspondent à deux ou plusieurs lignes espacées ou à une ligne et un ou plusieurs points espacés. Il est possible notamment d'utiliser des motifs adhésifs dans lesquels les zones adhésives forment une forme anisotrope, tels que les motifs adhésifs décrits notamment dans le brevet EP 1664266 auquel on pourra se référer pour plus de détails, ou tels que présentés sur les **Figures 4** ou **5****.**

Par « zone adhésive », on entend une zone cytophile, sur laquelle une cellule animale ou humaine va pouvoir adhérer, par adsorption, en particulier lorsque celle-ci est mise en contact avec le support dans un milieu de culture aqueux approprié, à 37°C et en l'absence de lumière. Une zone adhésive est une zone sur laquelle les protéines de la matrice extracellulaire (fibronectine, laminine, collagène...) sécrétées par les cellules durant la culture peuvent s'adsorber pour ensuite permettre l'adhésion des cellules via les intégrines (protéines transmembranaires des cellules). En particulier, les supports de verre, verre silanisé, silice ou de silicium oxydé en surface présentent des propriétés adhésives pour les cellules. Par ailleurs, de nombreuses molécules sont connues pour favoriser l'immobilisation de cellules. On peut citer les antigènes, les anticorps, les molécules d'adhésion cellulaire, les molécules de matrices extracellulaires tels que la laminine, la fibronectine et le collagène, les peptides synthétiques, les hydrates de carbone et analogues. Le choix des matériaux et/ou molécules présentes sur ces zones sera adapté, par l'homme du métier, aux cellules que l'on souhaite faire adhérer.

Par « zone non adhésive », on entend une zone cytophobe sur laquelle les cellules n'adhèrent pas, en particulier lorsque celle-ci est mise en contact avec le support dans un milieu de culture aqueux approprié, à 37°C et en l'absence de lumière. L'absence d'adhésion cellulaire sur cette zone est associée au fait que les protéines de la matrice extracellulaire (MEC) mentionnées plus haut ne peuvent ni s'y adsorber de façon spécifique ni s'y insérer. Le polymère assurant la fonction anti-adhésive est donc choisi de façon à ne présenter aucune interaction spécifique avec les protéines de la MEC : on préfèrera un polymère électriquement neutre, et ne présentant pas de séquences d'acides aminés reconnaissables par les protéines. Les cellules qui peuvent adhérer sur un support selon l'invention peuvent être tout type de cellule animale ou humaine. Il peut notamment s'agir de fibroblastes, de cellules hématopoïétiques, endothéliales ou épithéliales. La cellule peut être de type sauvage ou modifié. Il peut s'agir de cellules tumorales.

De manière préférée, le ou les motifs adhésifs utilisés dans le cadre de l'invention présente(nt) une taille qui est adaptée pour permettre l'adhésion d'une cellule individuelle unique. En particulier, les motifs adhésifs se présentent sous la forme de micro-motifs qui ont une taille qui correspond sensiblement à la taille de la cellule à immobiliser ou une taille telle qu'une seule cellule puisse s'y étaler et se diviser, avec un mouvement limité. Typiquement, la surface des motifs qui sont délimités par les zones adhésives dans le cas où elles sont toutes connectées ou par l'enveloppe reliant les zones adhésives lorsque celles-ci ne sont pas connectées, est de 1 à 2500 µm², de préférence de 1 à 2500 µm², notamment de 1 à 500 µm² ou de 500 à 900 µm². Par exemple, la largeur des lignes, ponts ou formes diverses constituant les zones adhésives seront de 1 à 20 µm, notamment de l'ordre de 10 µm.

Dans le cadre de l'invention, le rapport entre la zone non adhésive sur la surface totale du motif est, de préférence, dans la gamme allant de 30 à 90 % et préférentiellement entre 50 et 90 %.

Le plus souvent, un réseau de motifs adhésifs identiques tels que définis dans le cadre de l'invention sera créé à la surface du support. Le support présente donc une surface micro-structurée. De préférence, les motifs adhésifs sont régulièrement répartis et espacés dans le réseau, tel qu'illustré notamment **Figures 4** et **5****.** Un tel réseau va permettre l'adhésion de cellules individuelles à des emplacements bien déterminés au niveau des motifs adhésifs, empêchant ainsi la migration des cellules. De manière avantageuse, les motifs correspondront à des motifs tels que décrits dans le brevet EP 1664266 qui conduit à une polarisation des cellules qui viennent se placer de manière reproductible sur les motifs adhésifs. Lorsque le support comporte un réseau de motifs adhésifs, ces derniers sont isolés les uns des autres par des régions non-adhésives. De préférence, la distance moyenne séparant deux motifs adhésifs consécutifs sera au moins égale à 2 fois la distance maximale créée par la zone non adhésive et séparant deux zones adhésives d'un même motif. Le plus souvent, le polymère non-adhésif immobilisé sur les zones non adhésives des motifs adhésifs est également immobilisé sur les régions non adhésives séparant deux motifs adhésifs consécutifs. Le plus souvent, le support est, dans ce cas, totalement recouvert d'un polymère sélectionné de manière à présenter une conformation qui peut être modifiée par variation d'un paramètre de l'environnement dans lequel se trouve le support, tel que défini dans le cadre de l'invention, à l'exception des zones adhésives des motifs adhésifs.

A titre d'exemple, un réseau comprenant de 5 à 10 000 motifs adhésifs peut être réalisé à la surface du support. Il est, notamment, possible que 10 à 50 000 motifs adhésifs, et de préférence 5 000 à 15 000 adhésifs, soient présents par cm² de support.

Dans le cadre de l'invention, il est prévu d'immobiliser sur la zone non-adhésive du ou des motifs présents sur le support, un polymère sur lequel une cellule vivante ne va pas adhérer mais dont la conformation et donc la taille vont pouvoir être modifiées par modification d'un paramètre externe. En particulier, le polymère sélectionné va présenter une conformation et une taille donnée, dans les conditions d'adhésion et de culture de la cellule, qui vont pouvoir être modifiées pour obtenir un déploiement ou un gonflement du polymère, suite à la modification d'un paramètre externe. En général, l'adhésion et la culture cellulaire sont réalisées dans un milieu aqueux, à 37°C. Aussi, à titre de référence, on peut prendre la taille (c'est-à-dire la longueur que présente le polymère dans sa conformation) et la conformation du polymère dans l'eau pure à une température de 37°C. Le polymère immobilisé sur les zones non adhésives est apte à subir une modification de sa conformation entrainant une augmentation de sa taille, lorsque l'on fait varier un paramètre externe, également nommé environnemental, tels que la température ou l'éclairement. Cette augmentation de la taille du polymère va pousser la cellule, au niveau du pont qu'elle forme au-dessus du polymère et la contraindre à se détacher des zones adhésives.

Le polymère non-adhésif peut être un homopolymère ou un copolymère dont la solubilité dans l'eau dépend d'un paramètre environnemental externe. Dans les conditions environnementales usuelles pour la culture cellulaire, c'est-à-dire à une température de 37°C et en l'absence de lumière, l'eau pure doit être un mauvais solvant pour le polymère immobilisé sur le support, qui adopte alors une conformation dite globulaire ou compactée. Le détachement des cellules cultivées sur le support est induit par le changement de conformation du polymère, qui passe de sa forme globulaire (mauvais solvant) à une conformation gonflée (bon solvant) lors de la variation d'un paramètre environnemental.

Pour assurer l'innocuité de la variation d'environnement vis-à-vis des cellules cultivées, on préfèrera utiliser soit la température, soit une radiation électromagnétique, correspondant de préférence à une radiation lumineuse proche du visible, comme paramètre de contrôle de la conformation du polymère.

Le paramètre environnemental qui va être modifié peut, par exemple, correspondre à une variation de la température et en particulier à une diminution de la température. En particulier, le polymère non-adhésif peut être un polymère thermosensible présentant dans l'eau une température critique inférieure de solubilité (TCIS) qui appartient à la gamme allant de 25 à 35°C. La TCIS peut notamment être déterminée par une mesure, en fonction de la température, de l'absorption de la lumière par une solution diluée du polymère dans l'eau à une concentration pouvant aller de 0,01 à 1% en masse, la TCIS correspondant à l'augmentation de turbidité de la solution, et est définie comme la température à laquelle la transmission de la solution passe de 100% à 50%, comme décrit dans la publication de T. BALTES et al. Journal of Polymer Science Part A, 37 (1999) pp 2977-2989. A titre d'exemple de tels polymères, on peut citer le poly(N-isopropylacrylamide), le poly(oligo(éthylène glycol)méthacrylate) et le poly(N,N-diméthyl-aminoéthyl méthacrylate). Avec de tels polymères, le décollement des cellules est induit lorsque la température du milieu de culture dans lequel est disposé le support est abaissée de 37°C à une température inférieure à la TCIS.

Il est également possible que la variation du paramètre environnemental corresponde à une variation de l'éclairement lumineux à laquelle est soumis le support. Il est, en particulier, possible d'utiliser un polymère photostimulable, dont la conformation dans l'eau à 37°C passe d'une conformation que l'on nommera globulaire ou compactée en l'absence de lumière, à une conformation dite gonflée ou déployée, lorsque celui-ci est éclairé à une longueur d'onde particulière. Par exemple, le polymère non-adhésif peut être le poly{N,N-diméthylaminoéthylméthacrylate-co-4-méthyl-[7-(méthacryloyl)oxyéthyloxy]coumarin} qui présente une telle propriété lorsqu'il est éclairé à une longueur d'onde de 365nm.

De manière préférée, dans le cadre de l'invention, le polymère non adhésif est immobilisé sur la surface du support par liaison covalente. Ceci permet notamment de garantir la stabilité de la liaison polymère-support, notamment lors de la culture cellulaire. En particulier, le polymère non adhésif peut être immobilisé selon une brosse de chaînes polymériques, chacune des chaines polymériques étant liée à la surface du support par une de ses extrémités.

Le polymère non-adhésif forme une couche ou revêtement non adhésif pour les cellules, dont l'épaisseur peut être modifiée en jouant sur un paramètre environnemental. Dans le cadre de l'invention, il est préférable que le polymère non adhésif soit immobilisé avec une densité de chaines polymériques appartenant à la gamme allant de 0,1 à 1 chaîne par nm², ce qui permet d'optimiser son pouvoir adhésif. La présence du polymère sous forme d'une brosse d'une telle densité suffisamment élevée permet par ailleurs d'empêcher, du fait de la répulsion stérique, l'insertion des protéines dans le revêtement anti-adhésif.

De préférence, le changement de conformation du polymère, de sa conformation de référence dans l'eau à 37 °C en l'absence de lumière, ou de sa conformation compactée dans les conditions d'adhésion, à sa conformation déployée obtenue après variation du paramètre environnemental, est associé à une variation d'épaisseur moyenne de la couche formée par le polymère d'au moins un facteur 2. La variation d'épaisseur moyenne de cette couche, et en particulier de cette couche formée d'une brosse de polymères, entre la conformation compactée et la conformation déployée peut se caractériser, par exemple, par une mesure optique de l'épaisseur moyenne, par ellipsométrie en phase liquide. Par exemple, on peut utiliser un polymère non adhésif qui se présente sous la forme de chaines polymériques comprenant de 50 à 10000 unités monomériques.

Les supports utilisés se présentent, en général, sous la forme de plaque, lame ou lamelle, classiquement utilisés en tant que support d'analyse. Ils seront, par exemple, constitués de verre, verre silanisé, silice ou silicium oxydé en surface et comporteront des revêtements de surface comme expliqué précédemment et ci-après pour la constitution des motifs adhésifs. De tels supports, même en l'absence de revêtement sont adhésifs pour les cellules. Mais, il est possible que des molécules choisies parmi la laminine, la fibronectine et le collagène soient immobilisées sur les zones adhésives du motif adhésif.

La présente invention a également pour objet un procédé de préparation d'un support conforme à l'invention, dans lequel on immobilise, notamment par liaison covalente, le polymère non-adhésif sélectionné, sur la surface totale du support et ensuite on réalise par photolithographie, une photo-ablation du polymère non-adhésif au niveau des zones correspondant aux zones adhésives des motifs adhésifs. De manière avantageuse, l'immobilisation du polymère à la surface peut se faire par polymérisation radicalaire contrôlée d'au moins un monomère sur un initiateur de polymérisation immobilisé, de préférence par liaison covalente, à la surface du support.

Le revêtement anti-adhésif ainsi obtenu est formé d'une couche mince de polymère. Pour assurer la stabilité du revêtement, on préférera une couche liée de façon covalente au support, par exemple sous la forme d'une brosse dense de chaines polymériques. La liaison covalente se fait, en général, par couplage sur des fonctions Si-OH présentes en surface des supports utilisés de composés du type alcoxysilane (par exemple triméthoxy ou triéthoxysilane) ou halogénosilane (par exemple trichlorosilane) selon des techniques bien connues de l'homme de l'art. Cette brosse peut, par exemple, être élaborée par le biais de différentes techniques :
(i) par greffage de chaînes polymériques portant une fonction chimique terminale susceptible de former une liaison covalente avec un groupement préalablement greffé de façon covalente sur le support. Un exemple de cette méthode dite du « grafting-onto » consiste à greffer sur le support un silane exposant à la surface une liaison Si-H, puis de mettre en contact cette surface avec une solution ou un fondu de chaînes polymériques présentant une fonction terminale vinyl, en présence de platine agissant comme catalyseur. La réaction d'hydrosilylation qui se produit entre les Si-H de surface et les liaisons insaturées en bouts de chaînes permet de lier de façon covalente les macromolécules au support (Bureau et Leger, Langmuir 20 (2004) pp 4523-4529).
(ii) par polymérisation initiée à partir de la surface (méthode dite du « grafting-from »). La première étape de ce procédé consiste à greffer sur le support une molécule servant d'initiateur de la polymérisation. La nature de cette molécule dépend de la méthode de polymérisation choisie. Il peut s'agir d'un silane présentant la fonction terminale adaptée à l'amorçage de la polymérisation (par exemple une liaison C-X, ou C est un atome de carbone et X est un halogène comme le chlore, le brome ou l'iode, si la polymérisation se fait par polymérisation radicalaire par transfert atomique nommée ATRP). Ce greffage se fait typiquement en immergeant le support en verre ou en silice durant quelques minutes à quelques heures dans une solution diluée du silane dans un solvant organique (par exemple toluène, THF, cyclohexane) à une température pouvant varier de la température ambiante à environ 100°C. Alternativement, un silane présentant un groupe terminal amine (NH₂) peut être greffé en solution aqueuse sur le support. Le groupement amine est ensuite fonctionnalisé par réaction avec une molécule présentant le groupement servant d'initiateur de polymérisation, comme décrit dans l'exemple détaillé plus loin. La croissance des chaînes polymériques est ensuite réalisée, à partir de cet initiateur, par le biais d'une méthode de polymérisation radicalaire contrôlée telle que l'ATRP, par addition/fragmentation réversible par transfert de chaîne (RAFT), ou par polymérisation médiée par un radical nitroxyde ou un iniferter. Ces différentes techniques sont bien connues de l'homme du métier et sont décrites en détail par Barbey et al. dans la publication de Chem. Rev. 109, 5437-5527 (2009) à laquelle on pourra se référer pour plus de détails. La réaction de polymérisation est conduite en exposant le support portant l'amorceur à une solution du ou des monomères choisis, en présence des agents nécessaires à la polymérisation (catalyseur, ou agent RAFT par exemple).

La méthode décrite en (ii) sera préférée car elle permet l'élaboration de brosses de chaines polymériques de taille contrôlée et dont la densité de greffage (nombre de chaines polymériques par unité de surface) est élevée (typiquement de l'ordre de 0,1 à 1 chaîne par nm²), assurant ainsi de bonnes propriétés anti-adhésives.

Les conditions de polymérisation, et en particulier le temps de polymérisation et la concentration en molécules greffées en surface (polymère dans le cas (i) et initiateur de polymérisation dans le cas (ii)), seront adaptées par l'homme du métier en fonction de la densité et de l'épaisseur de la couche de polymère désirées.

Ensuite, à partir d'un revêtement de polymère obtenu selon les techniques ci-dessus qui couvre uniformément la surface du support, le revêtement de polymère est exposé, au travers d'un masque de photolithogravure portant les motifs souhaités, à un rayonnement ultra-violet dans une gamme de longueur d'onde, le plus souvent, inférieure à 200 nm (UV profonds). Ceci mène à la photo-ablation du polymère au niveau des zones du masque transparentes aux UV. Une exposition de quelques minutes conduit à l'ablation totale du polymère, ce qui permet de remettre à nu le support sous-jacent, sur lequel les cellules adhèrent de par la nature du matériau qui le constitue.

Bien que le procédé ci-dessus soit préféré, les motifs adhésifs sur le support peuvent être réalisés par toutes techniques permettant d'obtenir une surface sur laquelle des zones adhésives sont séparées par des régions couvertes du revêtement polymère anti-adhésif. Il est notamment possible d'utiliser l'impression par microcontact, afin de déposer sur le support la molécule servant d'initiateur à la polymérisation selon un motif négatif du motif adhésif final souhaité. La croissance des macromolécules est ensuite limitée aux zones de la surface fonctionnalisées lors du tamponnage, laissant par ailleurs le support non traité. Néanmoins, ce procédé n'est pas préféré car il présente les désavantages de la technique d'impression par microcontact détaillés en relation avec l'art antérieur.

Quelle que soit la technique utilisée pour greffer le polymère non adhésif aux endroits désirés, il est possible d'ajuster la spécificité de l'adhésion des zones adhésives du support en adsorbant dans les zones non couvertes de polymère non-adhésif, un ligand impliqué dans l'adhésion cellulaire, par exemple une protéine telle que la fibronectine, le collagène ou la laminine. En particulier, dans le cas du procédé détaillé en (i), des molécules choisies parmi la laminine, la fibronectine et le collagène seront immobilisées au niveau des zones adhésives, après photo-ablation du polymère non-adhésif. Cette étape peut être réalisée classiquement en mettant en contact, pendant une durée de l'ordre d'une heure, le support avec une solution aqueuse tamponnée du ligand souhaité, à une concentration allant typiquement de 0,1 à 100 µg/mL.

Les supports selon l'invention pourront être utilisés dans différentes applications en biologie cellulaire qui nécessitent d'immobiliser une ou des cellules sur un support, telles que la culture de cellules, la cytométrie, le criblage de médicament, la toxicologie, le diagnostic.

La présente invention a également pour objet un procédé d'analyse *in vitro* d'au moins une cellule comprenant les étapes suivantes :
- adhésion d'une cellule sur un motif adhésif d'un support selon l'invention,
- culture de ladite cellule,
- analyse par imagerie optique de ladite cellule adhérée sur le support.

Un tel procédé comprend, avantageusement, après l'étape d'analyse le détachement de la cellule par variation d'un paramètre environnemental entraînant un déploiement du polymère non-adhésif. La variation utilisée correspond, de préférence, à une variation de température ou d'éclairement lumineux précédemment mentionnées et sera adaptée aux polymères utilisées.

Les supports selon l'invention présentent l'avantage d'être réutilisables, Le processus de détachement des cellules n'altère pas le support qui présente une très bonne stabilité. L'adhésion par adsorption des cellules sur les zones adhésives est réversible ce qui est un avantage certain, par rapport aux techniques antérieures.

L'analyse par imagerie optique pourra utilisée toute technique automatisée, et notamment les techniques d'épifluorescence, telles que notamment décrites dans la demande EP 1664266.

Les cellules peuvent être cultivées directement sur les supports selon l'invention. La culture de cellules sur les supports obéit aux protocoles de biologie cellulaire standards : il convient en particulier d'adapter le milieu de culture au type cellulaire étudié. On pourra, par exemple, utiliser un milieu Eagle modifié par Dulbecco (DMEM) contenant 10 % de sérum foetal bovin, et 0,2% de pénicilline et de streptomycine. La culture peut être réalisée dans une atmosphère humidifiée contenant 5% de dioxyde de carbone gazeux et 95% d'air. L'ensemencement des cellules pourra se faire à une densité allant de 5000 à 50000 cellules/cm². Cette densité sera de préférence adaptée à la densité des motifs sur le support (par exemple, 10000 cellules par cm² pour une distance entre motifs de 100 µm). Le plus souvent, l'adhésion et la culture des cellules est réalisées dans un milieu de culture aqueux, à 37°C, en l'absence de lumière. La culture cellulaire peut être réalisée en présence d'un médicament ou molécule dont on souhaite tester l'activité.

Les exemples ci-après permettent d'illustrer l'invention mais n'ont aucun caractère limitatif.

L'élaboration de brosses de poly(N-isopropylacrylamide) (PNIPAM) greffées sur des supports de verre ou de silicium oxydé, et dans lesquelles des motifs adhésifs sont réalisés par photo-ablation aux UV profonds est décrite de manière détaillée. Ensuite, seront donnés les résultats obtenus concernant :
- la culture et l'adhésion de cellules sur ces surfaces,
- un exemple de mise en oeuvre d'analyse statistique relatif à la localisation spatiale d'une protéine impliquée dans l'adhésion cellulaire,
- le décollement des cellules du support, induit par une variation de température.

### Méthodes et instruments de caractérisation

Les observations de cellules ont été réalisées sur un microscope Nikon Ti-E équipé d'une enceinte d'incubation régulée en température, permettant de faire de l'imagerie en transmission, par épifluorescence ou en champ clair. Les images de la morphologie des cellules en microscopie par contraste de phase ont été obtenues sur un microscope Olympus IX71 avec un objectif 4x ou 10x, immédiatement à la sortie de l'incubateur ou bien après fixation des cellules au paraformaldéhyde. Les expériences de décollement de cellules induit par la température ont été réalisées sur le microscope Nikon ci-dessus, en fixant la température de l'enceinte climatique à 30°C et en suivant la morphologie des cellules au cours du temps.

Les brosses de PNIPAM élaborées sur wafer de silicium ont été caractérisées par la mesure de leur épaisseur sèche, à l'aide d'un ellipsomètre monochromatique (628nm) à lame quart d'onde tournante.

### Elaboration et caractérisation des brosses

Des brosses de poly(N-isopropylacrylamide) (PNIPAM) ont été greffées sur des supports en verre (lamelles couvre-objet) et des surfaces oxydées de supports (en anglais « wafers ») de silicium, par la technique de polymérisation radicalaire par transfert atomique, selon un protocole inspiré de celui décrit dans l'article de Malham et Bureau (Langmuir 26, 4762-4768 (2010)). Les échantillons obtenus sur wafer de silicium ont été utilisés pour la caractérisation par ellipsométrie de l'épaisseur des brosses. Les brosses déposées sur lamelles de verre ont été utilisées pour les expériences de microscopie sur les cultures de cellules réalisées.

*Réactifs:* Le monomère N-isopropylacrylamide a été purifié par recristallisation dans l'hexane afin d'éliminer l'inhibiteur de polymérisation qui le stabilise lors de sa commercialisation. Les autres réactifs et solvant, en particulier le 3-aminopropyl-triéthoxysilane (APTES), la triéthylamine (TEA), le chlorure de cuivre (CuCl), le 1,1,7,7-pentaméthyldiéthylenetriamine (PMDETA) et le bromure de 2-bromo-2-méthylpropionyl (BMPB) ont été obtenus en qualité pure ou ultra-pure (>98%) et utilisés tels quels. Toutes les solutions aqueuses ont été préparées avec de l'eau ultra-pure.

Les supports de verre et de silicium ont d'abord été nettoyés par rinçages successifs à l'acétone, à l'éthanol et à l'eau, puis immersion pendant 15 minutes dans une solution aqueuse d'hydroxyde de sodium de concentration 1M, et finalement rincés à l'eau.

Les supports ont ensuite été immergés dans une solution aqueuse d'APTES à une concentration de 2.10⁻⁴ M pendant 1 minute. La solution d'APTES a été préalablement préparée et agitée pendant une à deux heures avant l'immersion des échantillons, afin d'hydrolyser les groupements éthoxysilane de l'APTES en vue du greffage sur les surfaces. Cette étape permet de former sur les supports une couche exposant des fonctions NH₂ à la surface. La variation de la concentration en APTES ou du temps d'immersion affecte la densité surfacique de ces groupes NH₂, ce qui fixe la densité finale de la brosse de polymère. Les conditions mentionnées ci-dessus ont été identifiées comme permettant l'obtention d'une densité suffisante pour assurer le caractère anti-adhésif recherché, quelle que soit la température. Il a par ailleurs été constaté qu'avec une brosse élaborée à partir d'une solution de concentration d'APTES de 2.10⁻⁵ M, la densité obtenue, de l'ordre de 0,05 chaîne/nm², ne permet pas d'assurer la fonction anti-adhésive.

Les supports ont ensuite été mis en contact pendant une minute avec une solution de BMPB (250µL) dans le dichlorométhane (20 mL) en présence de TEA (1,2 mL). Cette étape permet de fonctionnaliser le groupe NH₂ en y greffant les molécules de BMBP, qui exposent à la surface les fonctions C-Br servant d'amorceur à la réaction de polymérisation du NIPAM.

Une solution de NIPAM (1g) et PMDETA (150µL) dans l'eau (20mL) a été préparée dans un ballon fermé par un bouchon septum et purgée de l'oxygène dissous par bullage d'argon pendant 30 minutes. 25mg de CuCl ont ensuite été ajoutés, puis la solution a été agitée durant quelques minutes jusqu'à l'obtention d'une solution homogène de couleur verte. Les supports ont ensuite été mis en contact, à température ambiante, avec cette solution pendant un temps variant de 30 secondes à 10 minutes, puis rincés abondamment à l'eau avant d'être séchés dans un flux d'azote gazeux. Ce temps d'immersion correspond à la durée de polymérisation, qui fixe le nombre de monomères par chaîne, et donc leur longueur.

Les brosses élaborées dans ces conditions sur wafer de silicium oxydé ont été caractérisées en mesurant, par ellipsométrie, leur épaisseur à sec, c'est-à-dire une fois débarrassées du solvant utilisé lors du greffage. La **Figure 2** montre l'augmentation de cette épaisseur à sec avec le temps de polymérisation, qui traduit l'augmentation de la longueur des macromolécules greffées. Toutes les brosses élaborées dans la gamme d'épaisseur figurant sur la **Figure 2** (entre 15 et 65 nm d'épaisseur) ont démontré d'excellentes propriétés anti-adhésives.

### Réalisation des motifs adhésifs

Les motifs adhésifs sur les brosses de PNIPAM ont été réalisés par photo-ablation aux UV profonds. Le dispositif d'illumination utilisé consiste en quatre lampes à basse pression de mercure (Heraeus NobleLight NIQ 60/35XL, 60W, rayonnement de longueur d'onde <200nm) disposées côte à côte horizontalement dans une enceinte en acier inoxydable. Les échantillons ont tous été insolés à une distance verticale de 9 cm des lampes.

Dans ces conditions, la cinétique de photo-ablation a tout d'abord été caractérisée en suivant, par ellipsométrie, l'évolution de l'épaisseur sèche d'une brosse greffée sur wafer de silicium en fonction du temps d'insolation. La **Figure 3** montre un exemple de cette évolution pour une brosse d'épaisseur initiale 65 nm. On constate qu'après 5 minutes d'illumination, la brosse a été totalement retirée du support.

Les brosses sèches de PNIPAM ont été mises en contact direct avec un photo-masque portant des motifs transparents de différentes géométries (annulaires, triangulaires, rectangulaires ou en V). Ces masques en quartz/chrome ont été obtenus auprès de Toppan Photomasks Inc. Texas USA. Le temps d'insolation a été choisi entre 5 et 10 minutes, sans que ce choix n'affecte de façon significative la qualité des motifs.

### Adsorption de protéines

Afin de révéler la forme et la définition des motifs réalisés, des expériences d'adsorption d'une protéine fluorescente ont été conduites. Une solution contenant 20 µg/mL de fibronectine/fibrinogène-Alexa fluor 546 nm (Invitrogen) dans une solution tampon à 10mM d'Hepes (pH 8,5) a été préparée. Les supports précédemment préparés portant les brosses de PNIPAM micro-structurées ont été mis en contact avec cette solution et incubés pendant une heure, à l'abri de la lumière, puis rincés avec une solution tampon saline de PBS (Phosphate Buffered Saline). Les **Figures 4** et **5** donnent des exemples de motifs réalisés, visualisés par microscopie par épifluorescence en détectant le signal provenant des protéines adsorbées. Ces figures démontrent :
(i) les bonnes propriétés anti-adhésives des brosses de PNIPAM (aucune fluorescence détectée en dehors des motifs adhésifs)
(ii) l'homogénéité des motifs à grande échelle (de l'ordre du mm²) obtenue avec la méthode décrite.
(iii) la bonne résolution et la variété de forme des motifs.

### Culture de cellules

Des cellules (fibroblastes) de type REF52 et MEF ont été cultivées, sur les supports élaborés. , en atmosphère humidifiée contenant 5% de dioxyde de carbone gazeux et 95% d'air, dans un milieu de Eagle modifié par Dulbecco (DMEM) contenant 10 % de sérum foetal bovin, et 0,2% de pénicilline et de streptomycine. Les REF52 ont été cultivées sur tous les supports élaborés. Les MEF ont été cultivées uniquement sur des supports revêtus d'une brosse de PNIPAM d'épaisseur sèche de 65 nm et de densité 0,5 chaine/nm². Les cellules ont été ensemencées sur les surfaces à une densité de 50000 cellules/cm². Après 30 minutes, les cellules non adhérentes situées entre les motifs ont été enlevées par rinçage avec du milieu de culture pur.

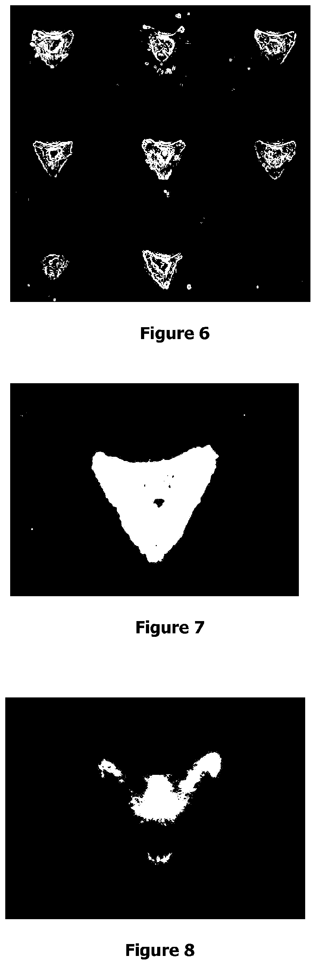

Les cellules ont été ensemencées soit sur les surfaces micro-structurées n'ayant subi aucun traitement préalable, soit sur des supports sur lesquels de la fibronectine/fibrinogène-alexa avait été pré-déposée. Un bon étalement des cellules sur les motifs a été observé dans les deux cas (voir **Figures 6** et **9**), avec des différences sur le temps nécessaire à obtenir cet étalement : 2 heures de culture ont été suffisantes sur les supports pré-revêtus de fibronectine, alors qu'une dizaine d'heures ont été nécessaires dans le cas d'un ensemencement sur les supports exposant la surface de verre nue dans les zones adhésives.

Après étalement des cellules, celles-ci ont été fixées au paraformaldéhyde.

### Résultats obtenus

### 1- Analyse statistique des réponses cellulaires

La **Figure 6** montre un exemple, obtenu par microscopie optique en transmission, d'un réseau formé par des cellules REF52 adhérées sur un motif en forme de V. Les REF52 sont des cellules exprimant une forme fluorescente de la paxilline (paxilline-YFP), une protéine associée au mécanisme d'adhésion cellule/support. Il a été montré dans des travaux antérieurs que lors de l'adhésion cellulaire sur un motif présentant des sommets, différentes protéines intracellulaires impliquées dans les interactions avec le support s'agrègent au niveau de ces sommets (Théry et al., Proc. Natl. Acad. Sci. USA. 103 (2006) pp 19771-19776). Nous avons pu mettre en évidence ce phénomène d'agrégation avec la paxilline en construisant l'image de fluorescence moyenne obtenue en superposant les images de 11 cellules différentes adhérées sur un motif en forme de V (**Figure 8**). L'intérêt de la construction de cette image moyenne et d'augmenter le rapport signal/bruit afin de mettre de évidence la localisation spatiale de la protéine, localisation qui n'apparaît pas ou peu sur l'image en fluorescence d'une cellule unique (**Figure 7**). Cet exemple de détermination de l'organisation intracellulaire montre que le support micro-structuré réalisé ici permet bien la mise en oeuvre d'une analyse statistique basée sur la reproductibilité de forme des cellules immobilisées sur la surface.

### 2- Obtention de différentes formes cellulaires

La méthode de photo-ablation décrite précédemment a été utilisée pour réaliser différentes formes de motifs adhésifs sur les surfaces de PNIPAM. La **Figure 9** montre trois exemples de motifs adhésifs (triangulaire, annulaire et rectangulaire) sur lesquels les cellules viennent adhérer en suivant la géométrie imposée par le support.

### 3- Décollement des cellules induit par une variation de température

Le décollement des cellules est induit lorsque la température est abaissée sous la TCIS du PNIPAM, qui vaut 32°C. Le mécanisme physique qui provoque le détachement des cellules est le changement de conformation des chaînes de PNIPAM greffées sur la surface lors de l'abaissement de température. Ce mécanisme est schématisé sur la **Figure 1**.

Les **Figures 10** et **11** montrent, pour deux formes de cellules initiales et pour les deux types cellulaires testés, une séquence temporelle d'images obtenues après diminution de la température de l'atmosphère à T=30°C. Ces séquences montrent la rétraction et l'arrondissement des cellules, ce qui indique leur décollement du support. Le décollement a lieu sur un temps allant de 25 à 45 minutes.

### 4- Stabilité des motifs - Possibilité de réutiliser les supports

Le détachement des cellules induit par la température permet non seulement de récupérer en solution la population des cellules étudiées, mais également de réutiliser le support micro-structuré pour réaliser d'autres cultures. La **Figure 12** montre des images correspondant à trois cultures successives, entre lesquelles les cellules ont été détachées par abaissement de la température jusqu'à la température ambiante et rinçage avec du milieu de culture pur. On constate que le taux d'occupation du réseau de motifs (nombre de cellules par unité de surface) est faiblement affecté entre la première culture et les deux suivantes (ce taux passe de 100 cellules/mm² à environ 80 cellules/mm² entre la première culture et les deux suivantes). Par ailleurs, la **Figure 12 (d)** montre qu'aucune cellule ne reste adhérée sur le support entre deux cultures.

La possibilité de réutiliser les supports pour plusieurs cultures démontre également la bonne stabilité des motifs adhésifs réalisés avec la méthode décrite dans cet exemple.

## Revendications

1. Support d'adhésion pour au moins une cellule animale ou humaine sur la surface duquel est créé un motif d'adhésion pour au moins une cellule animale ou humaine, ledit motif comportant au moins deux zones adhésives à ladite cellule qui sont espacées par une zone non adhésive à ladite cellule sur laquelle un polymère non-adhésif à ladite cellule est immobilisé, **caractérisé en ce que** les zones adhésives d'un motif adhésif sont disposées de manière telle que la cellule puisse adhérer, dans des conditions d'adhésion, sur les zones adhésives en formant un pont sur le polymère non-adhésif, le polymère présente une conformation qui peut être modifiée par variation d'un paramètre de l'environnement dans lequel se trouve le support et se présente sous une conformation dite compactée, dans les conditions d'adhésion de la cellule, pour se déployer dans une conformation dite déployée par modification d'un paramètre de l'environnement dans lequel se trouve le support.

2. Support selon la revendication 1 **caractérisé en ce que** le polymère définit une couche dont l'épaisseur peut être augmentée par rapport à une épaisseur de référence lorsque le support est placé dans l'eau pure à 37°C et en l'absence de lumière, par modification d'un paramètre de l'environnement dans lequel se trouve le support.

3. Support selon la revendication 1 ou 2 **caractérisé en ce que** le rapport entre la zone non adhésive sur la surface totale du motif est entre 50 et 90%.

4. Support selon la revendication 1, 2 ou 3 **caractérisé en ce que** le polymère non-adhésif forme une couche dont l'épaisseur moyenne peut être augmentée d'au moins un facteur 2, par rapport à son épaisseur moyenne de référence lorsque le support est placé dans l'eau pure à 37°C et en l'absence de lumière, ou par rapport à son épaisseur moyenne dans sa conformation compactée, grâce à la modification d'un paramètre de l'environnement dans lequel se trouve le support.

5. Support selon l'une des revendications 1 à 4 **caractérisé en ce que** le polymère présente une conformation qui peut être modifiée par variation de la température et en particulier par diminution de la température.

6. Support selon la revendication 5 **caractérisé en ce que** le polymère non-adhésif est un polymère thermosensible présentant dans l'eau une température critique inférieure de solubilité qui appartient à la gamme allant de 25 à 35°C.

7. Support selon la revendication 5 ou 6 **caractérisé en ce que** le polymère est choisi parmi le poly(N-isopropylacrylamide), le poly(oligo(éthylène glycol)méthacrylate) et le poly(N,N-diméthyl-aminoéthyl méthacrylate).

8. Support selon l'une des revendications 1 à 4 **caractérisé en ce que** le polymère présente une conformation qui peut être modifiée par variation de l'éclairement lumineux.

9. Support selon la revendication 8 **caractérisé en ce que** le polymère est le poly{N,N-diméthylaminoéthylméthacrylate-co-4-méthyl-[7-(méthacryloyl)oxyéthyloxy]coumarin}.

10. Support selon l'une des revendications précédentes **caractérisé en ce que** le polymère non adhésif est immobilisé sur la surface du support par liaison covalente.

11. Support selon l'une des revendications précédentes **caractérisé en ce que** le polymère non adhésif est immobilisé avec une densité de chaînes polymériques appartenant à la gamme allant de 0,1 à 1 chaînes par nm².

12. Support selon l'une des revendications précédentes **caractérisé en ce que** le polymère non adhésif est immobilisé selon une brosse de chaînes polymériques, chacune des chaînes polymériques étant liée à la surface du support par une de ses extrémités.

13. Support selon l'une des revendications précédentes **caractérisé en ce que** le polymère non adhésif se présente sous la forme de chaines polymériques comprenant de 50 à 10000 unités monomériques.

14. Support selon l'une des revendications précédentes **caractérisé en ce que** la taille du motif est adaptée pour permettre l'adhésion d'une cellule individuelle unique.

15. Support selon l'une des revendications précédentes **caractérisé en ce que** la distance maximale créée par la zone non adhésive séparant deux zones adhésives d'un motif, est inférieure à 50 µm, de préférence appartient à la gamme allant de 5 à 20 µm.

16. Support selon l'une des revendications précédentes **caractérisé en ce que** des molécules choisies parmi la laminine, la fibronectine et le collagène sont immobilisées sur les zones adhésives du motif adhésif.

17. Support selon l'une des revendications précédentes **caractérisé en ce que** les zones adhésives des motifs adhésifs sont connectées entre elles pour former une forme ouverte ou fermée ou sont isolées les unes des autres.

18. Support selon l'une des revendications précédentes **caractérisé en ce que** les zones adhésives des motifs adhésifs forment une ligne brisée ou courbe qui peut être fermée délimitant par exemple un polygone ou un anneau ou ouverte, par exemple sous la forme d'un V, U, C ou L, ou bien correspondent à deux lignes espacées ou à une ligne et un ou plusieurs points espacés.

19. Support selon l'une des revendications précédentes **caractérisé en ce que** un réseau de motifs adhésifs identiques tels que définis aux revendications précédentes est présents sur sa surface.

20. Support selon la revendication 19 **caractérisé en ce que** le polymère non-adhésif immobilisé sur les zones non adhésives des motifs adhésifs est également immobilisé sur les régions séparant deux motifs adhésifs consécutifs.

21. Support selon l'une des revendications précédentes **caractérisé en ce que** le support est constitué de verre, verre silanisé, silice ou silicium oxydé en surface.

22. Procédé de préparation d'un support selon l'une des revendications précédentes, dans lequel on immobilise, notamment par liaison covalente, le polymère non-adhésif sélectionné, sur la surface totale du support et ensuite on réalise par photolithographie, une photo-ablation du polymère non-adhésif au niveau des zones correspondant aux zones adhésives des motifs adhésifs.

23. Procédé de préparation selon la revendication 22 **caractérisé en ce que** l'immobilisation du polymère non-adhésif sélectionné à la surface du support est réalisée par polymérisation radicalaire contrôlée d'au moins un monomère sur un initiateur de polymérisation immobilisé, de préférence par liaison covalente, à la surface du support.

24. Procédé de préparation selon la revendication 22 ou 23 **caractérisé en ce que** des molécules choisies parmi la laminine, la fibronectine et le collagène sont immobilisées au niveau des zones adhésives, après photo-ablation du polymère non-adhésif.

25. Procédé de détachement *in vitro* d'au moins une cellule préalablement adhérée sur le ou les motifs adhésifs d'un support selon l'une des revendications 1 à 21 **caractérisé en ce que** le détachement de la cellule est réalisé par variation d'un paramètre environnemental entrainant un déploiement du polymère non-adhésif.

26. Procédé d'analyse *in vitro* d'au moins une cellule comprenant les étapes suivantes :
- adhésion d'une cellule sur un motif adhésif d'un support selon l'une des revendications 1 à 21,
- culture de ladite cellule,
- analyse par imagerie optique de ladite cellule adhérée sur le support.

27. Procédé d'analyse selon la revendication 26 **caractérisé en ce qu'**il comprend après l'étape d'analyse le détachement de la cellule par variation d'un paramètre environnemental entraînant un déploiement du polymère non-adhésif.

## Patentansprüche

1. Haftträger für wenigstens eine tierische oder menschliche Zelle, auf dessen Oberfläche ein Haftmuster für wenigstens eine tierische oder menschliche Zelle erzeugt wird, wobei das Muster wenigstens zwei an der Zelle haftende Bereiche umfasst, die durch einen an der Zelle nicht haftenden Bereich beabstandet sind, auf dem ein an der Zelle nicht haftendes Polymer festgelegt ist, **dadurch gekennzeichnet, dass** die Haftbereiche eines Haftmusters derart angeordnet sind, dass die Zelle unter Haftbedingungen, unter Ausbildung einer Brücke auf dem nicht haftenden Polymer, an den Haftbereichen haften kann, das Polymer eine Gestalt aufweist, die durch Änderung eines Parameters der Umgebung, in der sich der Träger befindet, verändert werden kann, und unter den Haftbedingungen der Zelle in einer sogenannten verdichteten Gestalt vorliegt, um sich durch Änderung eines Parameters der Umgebung, in der sich der Träger befindet, in eine sogenannte entfaltete Gestalt zu entfalten.

2. Träger nach Anspruch 1, **dadurch gekennzeichnet, dass** das Polymer eine Schicht definiert, deren Dicke gegenüber einer Referenzdicke, wenn der Träger in reinem Wasser bei 37 °C und in Abwesenheit von Licht angeordnet ist, durch Änderung eines Parameters der Umgebung, in der sich der Träger befindet, erhöht werden kann.

3. Träger nach Anspruch 1 oder 2, **dadurch gekennzeichnet, dass** das Verhältnis des nicht haftenden Bereichs zu der Gesamtfläche des Musters zwischen 50 et 90 % beträgt.

4. Träger nach Anspruch 1, 2 oder 3, **dadurch gekennzeichnet, dass** das nicht haftende Polymer eine Schicht bildet, deren mittlere Dicke gegenüber seiner mittleren Referenzdicke, wenn der Träger in reinem Wasser bei 37 °C und in Abwesenheit von Licht angeordnet ist, oder gegenüber seiner mittleren Dicke in seiner verdichteten Gestalt, durch die Änderung eines Parameters der Umgebung, in der sich der Träger befindet, wenigstens um einen Faktor 2 erhöht werden kann.

5. Träger nach einem der Ansprüche 1 bis 4, **dadurch gekennzeichnet, dass** das Polymer eine Gestalt aufweist, die durch Änderung der Temperatur und insbesondere durch Verringerung der Temperatur verändert werden kann.

6. Träger nach Anspruch 5, **dadurch gekennzeichnet, dass** das nicht haftende Polymer ein wärmeempfindliches Polymer ist, das in Wasser eine kritische untere Löslichkeitstemperatur im Bereich von 25 bis 35 °C aufweist.

7. Träger nach Anspruch 5 oder 6, **dadurch gekennzeichnet, dass** das Polymer aus Poly(N-isopropylacrylamid), Poly(oligo(ethylen glycol)methacrylat) und Poly(N,N-dimethyl-aminoethyl methacrylat) ausgewählt ist.

8. Träger nach einem der Ansprüche 1 bis 4, **dadurch gekennzeichnet, dass** das Polymer eine Gestalt aufweist, die durch Änderung der Beleuchtungsstärke verändert werden kann.

9. Träger nach Anspruch 8, **dadurch gekennzeichnet, dass** das Polymer Poly{N,N-dimethylaminoethylmethacrylat-co-4-methyl-[7-(methacryloyl)oxyethyloxy]cumarin} ist.

10. Träger nach einem der vorhergehenden Ansprüche, **dadurch gekennzeichnet, dass** das nicht haftende Polymer auf der Oberfläche des Trägers durch kovalente Bindung festgelegt ist.

11. Träger nach einem der vorhergehenden Ansprüche, **dadurch gekennzeichnet, dass** das nicht haftende Polymer mit einer im Bereich von 0,1 bis 1 Kette pro nm² liegenden Polymerkettendichte festgelegt ist.

12. Träger nach einem der vorhergehenden Ansprüche, **dadurch gekennzeichnet, dass** das nicht haftende Polymer entsprechend einer Polymerkettenbürste festgelegt ist, wobei eine jede der Polymerketten durch eine ihrer Enden mit der Oberfläche des Trägers verbunden ist.

13. Träger nach einem der vorhergehenden Ansprüche, **dadurch gekennzeichnet, dass** das nicht haftende Polymer in Form von Polymerketten mit 50 bis 10000 Monomereinheiten vorliegt.

14. Träger nach einem der vorhergehenden Ansprüche, **dadurch gekennzeichnet, dass** die Größe des Musters dazu ausgelegt ist, das Haften einer einzigen Einzelzelle zu ermöglichen.

15. Träger nach einem der vorhergehenden Ansprüche, **dadurch gekennzeichnet, dass** der maximale Abstand, der durch den nicht haftenden Bereich, welcher zwei Haftbereiche eines Musters trennt, erzeugt wird, weniger als 50 µm beträgt, vorzugsweise im Bereich von 5 bis 20 µm liegt.

16. Träger nach einem der vorhergehenden Ansprüche, **dadurch gekennzeichnet, dass** Moleküle, die aus Laminin, Fibronektin und Kollagen ausgewählt sind, auf den Haftbereichen des Haftmusters festgelegt sind.

17. Träger nach einem der vorhergehenden Ansprüche, **dadurch gekennzeichnet, dass** die Haftbereiche der Haftmuster untereinander verbunden sind, um eine offene oder geschlossene Form zu bilden, oder voneinander isoliert sind.

18. Träger nach einem der vorhergehenden Ansprüche, **dadurch gekennzeichnet, dass** die Haftbereiche der Haftmuster eine geknickte oder gekrümmte Linie bilden, die geschlossen sein kann, beispielsweise ein Vieleck oder einen Ring begrenzend, oder offen sein kann, beispielsweise in Form eines V, U, C oder L, oder aber zwei beabstandeten Linien oder einer Linie und einem oder mehreren beabstandeten Punkten entsprechen.

19. Träger nach einem der vorhergehenden Ansprüche, **dadurch gekennzeichnet, dass** ein Netz aus identischen Haftmustern, wie sie in den vorhergehenden Ansprüche definiert sind, auf seiner Oberfläche vorhanden ist.

20. Träger nach Anspruch 19, **dadurch gekennzeichnet, dass** das nicht haftende Polymer, das auf den nicht haftenden Bereichen der Haftmuster festgelegt ist, auch auf den Bereichen, die zwei aufeinander folgende Haftmuster trennen, festgelegt ist.

21. Träger nach einem der vorhergehenden Ansprüche, **dadurch gekennzeichnet, dass** der Träger aus Glas, silanisiertem Glas, Silizumdioxid oder oberflächenoxidiertem Silizium besteht.

22. Verfahren zur Herstellung eines Träger nach einem der vorhergehenden Ansprüche, bei dem insbesondere durch kovalente Bindung das ausgewählte nicht haftende Polymer auf der Gesamtfläche des Trägers festgelegt wird und anschließend durch Photolithographie eine Photoablation des nicht haftenden Polymers in Höhe der Bereiche, welche den haftenden Bereichen der Haftmuster entsprechen, durchgeführt wird.

23. Herstellungsverfahren nach Anspruch 22, **dadurch gekennzeichnet, dass** das Festlegen des ausgewählten nicht haftenden Polymers an der Oberfläche des Trägers mittels kontrollierter radikalischer Polymerisation wenigstens eines Monomers auf einem festgelegten Polymerisationsinitiator, vorzugsweise durch kovalente Bindung, an der Oberfläche des Trägers vollzogen wird.

24. Herstellungsverfahren nach Anspruch 22 oder 23, **dadurch gekennzeichnet, dass** Moleküle, die aus Laminin, Fibronektin und Kollagen ausgewählt sind, in Höhe der Haftbereiche nach Photoablation des nicht haftenden Polymers festgelegt werden.

25. Verfahren zum *in vitro*-Ablösen wenigstens einer Zelle, die zuvor auf dem oder den Haftmuster(n) eines Trägers nach einem der Ansprüche 1 bis 21 haftete, **dadurch gekennzeichnet, dass** das Ablösen der Zelle durch Änderung eines Umgebungsparameters, die zu einem Entfalten des nicht haftenden Polymers führt, vollzogen wird.

26. Verfahren zur *in vitro*-Analyse wenigstens einer Zelle, das die folgenden Schritte umfasst:
- Haften einer Zelle auf einem Haftmuster eines Trägers nach einem der Ansprüche 1 bis 21,
- Kultivieren der Zelle,
- Analyse der Zelle, die auf dem Träger haftete, durch optische Bildgebung.

27. Analyseverfahren nach Anspruch 26, **dadurch gekennzeichnet, dass** es nach dem Analyseschritt das Ablösen der Zelle durch Änderung eines Umgebungsparameters, die zu einem Entfalten des nicht haftenden Polymers führt, umfasst.

## Claims

1. A substrate for adhesion of at least one animal or human cell on the surface of which is generated an adhesion pattern for at least one animal or human cell, said pattern including at least two adhesive areas to said cell which are spaced from each other by a non-adhesive area to said cell on which a non-adhesive polymer to said cell is immobilized, **characterized in that** the adhesive areas of an adhesive pattern are placed so that the cell may adhere, under adhesion conditions, onto the adhesive areas by forming a bridge over the non-adhesive polymer, the polymer has a conformation which may be modified by changing a parameter of the environment in which the substrate is found and appears as a so-called compacted conformation, under adhesion conditions of the cell, in order to be deployed in a so-called deployed conformation by modification of a parameter of the environment in which the substrate is found.

2. The substrate according to claim 1, **characterized in that** the polymer defines a layer for which the thickness may be increased relatively to a reference thickness when the substrate is placed in pure water at 37°C and in the absence of light, by modification of a parameter of the environment in which the substrate is found.

3. The substrate according to claim 1 or 2, **characterized in that** the ratio between the non-adhesive area and the total surface area of the pattern is between 50 and 90%.

4. The substrate according to claim 1, 2 or 3, **characterized in that** the non-adhesive polymer forms a layer for which the average thickness may be increased by at least a factor of 2, relatively to its average reference thickness when the substrate is placed in pure water at 37°C and in the absence of light, or relatively to its average thickness in its compacted conformation, by means of the modification of a parameter of the environment in which the substrate is found.

5. The substrate according to one of claims 1 to 4, **characterized in that** the polymer has a conformation which may be modified by varying the temperature and in particular by lowering the temperature.

6. The substrate according to claim 5, **characterized in that** the non-adhesive polymer is a heat-sensitive polymer having in water a lower critical solubility temperature which belongs to the range from 25 to 35°C.

7. The substrate according to claim 5 or 6, **characterized in that** the polymer is selected from poly(N-isopropylacrylamide), poly(oligo(ethylene glycol)methacrylate) and poly(N,N-dimethyl-aminoethyl methacrylate).

8. The substrate according to one of claims 1 to 4, **characterized in that** the polymer has a conformation which may be modified by varying the light illumination.

9. The substrate according to claim 8, **characterized in that** the polymer is poly{N,N-dimethylaminoethylmethacrylate-co-4-méthyl-[7-(methacryloyl)oxyethyloxy]coumarin}.

10. The substrate according to one of the preceding claims, **characterized in that** the non-adhesive polymer is immobilized on the surface of the substrate through a covalent bond.

11. The substrate according to one of the preceding claims, **characterized in that** the non-adhesive polymer is immobilized with a density of polymeric chains belonging to the range from 0.1 to 1 chain per nm².

12. The substrate according to one of the preceding claims, **characterized in that** the non-adhesive polymer is immobilized according to a brush of polymeric chains, each of the polymeric chains being bound to the surface of the substrate through one of its ends.

13. The substrate according to one of the preceding claims, **characterized in that** the non-adhesive polymer appears in the form of polymeric chains comprising from 50 to 10,000 monomer units.

14. The substrate according to one of the preceding claims, **characterized in that** the size of the pattern is adapted so as to allow adhesion of a single individual cell.

15. The substrate according to one of the preceding claims **characterized in that** the maximum distance generated by the non-adhesive area separating two adhesive areas of a pattern, is less than 50 µm, preferably belongs to the range from 5 to 20 µm.

16. The substrate according to one of the preceding claims, **characterized in that** molecules selected from laminin, fibronectin and collagen are immobilized on the adhesive areas of the adhesive pattern.

17. The substrate according to one of the preceding claims, **characterized in that** the adhesive areas of the adhesive patterns are connected together in order to form an open or closed form or are isolated from each other.

18. The substrate according to one of the preceding claims, **characterized in that** the adhesive areas of the adhesive patterns form a broken line or curve which may be closed for example delimiting a polygon or a ring or open, for example with the shape of a V, U, C or L, or else correspond to two spaced apart lines or to a line and one or several spaced apart dots.

19. The substrate according to one of the preceding claims, **characterized in that** a network of identical adhesive patterns as defined in the preceding claims is present at its surface.

20. The substrate according to claim 19, **characterized in that** the non-adhesive polymer immobilized on the non-adhesive areas of the adhesive patterns is also immobilized on the regions separating two consecutive adhesive patterns.

21. The substrate according to one of the preceding claims, **characterized in that** the substrate consists of glass, silanized glass, silica or silicon oxidized at the surface.

22. A method for preparing a substrate according to one of the preceding claims, wherein the selected non-adhesive polymer is immobilized notably through a covalent bond, on the total surface of the substrate and photo-ablation of the non-adhesive polymer is then achieved by photolithography at the areas corresponding to the adhesive areas of the adhesive patterns.

23. The preparation method according to claim 22, **characterized in that** the immobilization of the selected non-adhesive polymer at the surface of the substrate is achieved by controlled radical polymerization of at least one monomer on an immobilized polymerization initiator, preferably through a covalent bond, at the surface of the substrate.

24. The preparation method according to claim 22 or 23, **characterized in that** molecules selected from laminin, fibronectin and collagen are immobilized at the adhesive areas after photo-ablation of the non-adhesive polymer.

25. A method for detachment *in vitro* of at least one cell having adhered beforehand on the adhesive pattern(s) of a substrate according to one of claims 1 to 21, **characterized in that** the detachment of the cell is achieved by varying an environmental parameter causing deployment of the non-adhesive polymer.

26. A method for *in vitro* analysis of at least one cell comprising the following steps:
- adhesion of a cell on an adhesive pattern of a substrate according to one of claims 1 to 21,
- cultivation of said cell,
- analysis by optical imaging of said cell adhered onto the substrate.

27. The analysis method according to claim 26, **characterized in that** it comprises, after the analysis step, detachment of the cell by varying an environmental parameter causing deployment of the non-adhesive polymer.
